# EUROPEAN PATENT APPLICATION

(11) **EP 4 549 577 A1**
(43) Date of publication of application: **07.05.2025**
(21) Application number: 23206834.6
(22) Date of filing: 30.10.2023
(51) Int. Cl.: C12N 15/85

(54) **NOVEL INDUCED CYTOPLASMIC IVT (ICIVT)-LIKE COMPOSITION AND THE RELATED VACCINE MEDICINE DESIGNS THEREOF**

(71) Applicant: Mello Biotech Taiwan Co., Ltd., Taipei City 115 (TW); Lin, Shi-Lung, Arcadia, CA 91006 (US); Lin, Sam, Arcadia, CA 91006 (US); Hung, William, Taipei City (TW)
(72) Inventor: LIN, Shi-Lung, Arcadia (US); LIN, Sam, Arcadia (US); HUNG, William, Taipei City (TW); LIU, Hsien-Lin, Taoyuan City (TW); WU, Yu-Jing, Taoyuan City (TW)
(74) Representative: Balder IP Law, S.L.

(57) **Abstract**

This invention generally relates to a novel induced cytoplasmic IVT (ICIVT) composition useful for inducing in-vitro transcription (IVT)-like RNA/mRNA amplification in the cells of interest after transfection in vitro, ex vivo and/or in vivo. The present invention is useful for designing and developing a variety of RNA/mRNA medicines as well as vaccines comprising a mixture of at least a promoter-linked RNA/mRNA-coding DNA (PLRcD) template and another DNA-dependent RNA polymerase (DdRP) mRNA sequence. Preferably, the DdRP mRNA may be selected from the mRNA of T7, T3 and/or SP6 RNA polymerase, or a combination thereof, while the PLRcD template may encode the transcripts of antisense RNA oligonucleotide (aRNA-ASO), small interferring RNA (siRNA), double-stranded RNA (dsRNA), short hairpin RNA (shRNA), microRNA (miRNA)/microRNA precursor (pre-miRNA), long noncoding RNA (IncRNA), messenger RNA (mRNA), and/or self-amplifying RNA/mRNA (saRNA/samRNA), or a combination thereof.

SEQUENCE LISTING

(1) GENERAL INFORMATION:
(iii) NUMBER OF SEQUENCES: 21

(2) INFORMATION FOR SEQ ID NO:1:
(i) SEQUENCE CHARACTERISTICS:
(A) LENGTH: 6 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION:
RCCRCC 6

(2) INFORMATION FOR SEQ ID NO:2:
(i) SEQUENCE CHARACTERISTICS:
(A) LENGTH: 6 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION:
RCCDCC 6

(2) INFORMATION FOR SEQ ID NO:3:
(i) SEQUENCE CHARACTERISTICS:
(A) LENGTH: 73 base pairs

(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:3:
GCUCCCUUCA ACUUUAACAU GGAAGUGCUU UCUGUGACUU
UAAAAGUAAG UGCUUCCAUG UUUUAGUAGG AGU 73

(2) INFORMATION FOR SEQ ID NO:4:
(i) SEQUENCE CHARACTERISTICS:
(A) LENGTH: 68 base pairs
(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:4:
CCUUUGCUUU AACAUGGGGG UACCUGCUGU GUGAAACAAA
AGUAAGUGCU UCCAUGUUUC AGUGGAGG 68

(2) INFORMATION FOR SEQ ID NO:5:
(i) SEQUENCE CHARACTERISTICS:
(A) LENGTH: 69 base pairs
(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:5:
CCACCACUUA AACGUGGAUG UACUUGCUUU GAAACUAAAG
AAGUAAGUGC UUCCAUGUUU UGGUGAUGG 69

(2) INFORMATION FOR SEQ ID NO:6:
(i) SEQUENCE CHARACTERISTICS:
(A) LENGTH: 68 base pairs
(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:6:
CCUCUACUUU AACAUGGAGG CACUUGCUGU GACAUGACAA
AAAUAAGUGC UUCCAUGUUU GAGUGUGG 68

(2) INFORMATION FOR SEQ ID NO:7:
(i) SEQUENCE CHARACTERISTICS:
(A) LENGTH: 44 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:7:
CUGUGUGGCU GUCACUCGGC UGCAUGCUUA GUGCACUCAC
GCAG 44

(2) INFORMATION FOR SEQ ID NO:8:
(i) SEQUENCE CHARACTERISTICS:
(A) LENGTH: 65 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:8:
CUGUGUGGCU GUCACUCGGC UGCAUGCUUA GUGCACUCAC
GCAGUAUAAU UAAUAACUAA UUACU 65

(2) INFORMATION FOR SEQ ID NO:9:
(i) SEQUENCE CHARACTERISTICS:
(A) LENGTH: 147 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:9:
GUCGUUGACA GGACACGAGU AACUCGUCUA UCUUCUGCAG
GCUGCUUACG GUUUCGUCCG UGUUGCAGCC GAUCAUCAGC
ACAUCUAGGU UUCGUCCGGG UGUGACCGAA AGGUAAGAUG
GAGAGCCUUG UCCCUGGUUU CAACGAG 147

(2) INFORMATION FOR SEQ ID NO:10:
(i) SEQUENCE CHARACTERISTICS:
(A) LENGTH: 88 base pairs
(B) TYPE: nucleic acid

(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:10:
AAUUAUAAAU UACCAGAUGA UUUUACAGGC UGCGUUAUAG
CUUGGAAUUC UAACAAUCUU GAUUCUAAGG UUGGUGGUAA
UUAUAAUU 88

(2) INFORMATION FOR SEQ ID NO:11:
(i) SEQUENCE CHARACTERISTICS:
(A) LENGTH: 129 base pairs
(B) TYPE: nucleic acid
(C) STRANDEDNESS: single
(D) TOPOLOGY: multiple stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:11:
CACAAAUAUU ACCAGAUCCA UCAAAACCAA GCAAGAGGUC
AUUUAUUGAA GAUCUACUUU UCAACAAAGU GACACUUGCA
GAUGCUGGCU UCAUCAAACA AUAUGGUGAU UGCCUUGGUG
AUAUUGCUG 129

(2) INFORMATION FOR SEQ ID NO:12:
(i) SEQUENCE CHARACTERISTICS:
(A) LENGTH: 189 base pairs
(B) TYPE: nucleic acid
(C) STRANDEDNESS: single
(D) TOPOLOGY: multiple stem-loop

(ii) MOLECULE TYPE: RNA

(A) DESCRIPTION: /desc = "synthetic"

(iii) HYPOTHETICAL: NO

(iv) ANTI-SENSE: NO

(xi) SEQUENCE DESCRIPTION: SEQ ID NO:12:
GCAAAAAUGU GAUCUUGCUU GUAAAUACAA UUUUGAGAGG
UUAAUAAAUU ACAAGUAGUG CUAUUUUUGU AUUUAGGUUA
GCUAUUUAGC UUUACGUUCC AGGAUGCCUA GUGGCAGCCC
CACAAUAUCC AGGAAGCCCU CUCUGCGGUU UUUCAGAUUC
GUUAGUCGAA AAACCUAAGA AAUUUAAUG 189

(2) INFORMATION FOR SEQ ID NO:13:
(i) SEQUENCE CHARACTERISTICS:
(A) LENGTH: 294 base pairs
(B) TYPE: nucleic acid
(C) STRANDEDNESS: single
(D) TOPOLOGY: multiple stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:13:
CACUCCCCUG UGAGGACUAC UGUCUUCACG CAGAAAGCGU
CUAGCCAUGG CGUUAGUAUG AGUGUCGUGC AGCCUCCAGG
ACCCCCCCUC CCGGGAGAGC CAUAGUGGUC UGCGGAACCG
GUGAGUACAC CGGAAUUGCC AGGACGACCG GGUCCUUUCU
UGGAUCAACC CGCUCAAUGC CUGGAGAUUU GGGCGUGCCC
CCGCGAGACU GCUAGCCGAG UAGUGUUGGG UCGCGAAAGG
CCUUGUGGUA CUGCCUGAUG GGUGCUUGCG AGUGCCCCGG
GAGGUCUCGU AGAC 294

(2) INFORMATION FOR SEQ ID NO:14:
(i) SEQUENCE CHARACTERISTICS:
(A) LENGTH: 55 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:14:
GGACACGAGU AACUCGUCUA UCUUCUGCAG GCUGCUUACG
GUUUCGUCCG UGUUG 55

(2) INFORMATION FOR SEQ ID NO:15:
(i) SEQUENCE CHARACTERISTICS:
(A) LENGTH: 52 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:15:
CAGCCGAUCA UCAGCACAUC UAGGUUUUGU CCGGGUGUGA
CCGAAAGGUA AG 52

(2) INFORMATION FOR SEQ ID NO:16:
(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: YES

(xi) SEQUENCE DESCRIPTION: SEQ ID NO:16:
CAGTTCCAAT TGTGAAGATT CTC 23

(2) INFORMATION FOR SEQ ID NO:17:
(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: YES

(xi) SEQUENCE DESCRIPTION: SEQ ID NO:17:
CTTGATGACG TTCTCAGTGC 20

(2) INFORMATION FOR SEQ ID NO:18:
(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:18:
GATATCTAAT ACGACTCACT ATAGGGAGAG GTGCCACCAT
GGTACTTGGT AGTT 54

(2) INFORMATION FOR SEQ ID NO:19:
(i) SEQUENCE CHARACTERISTICS:
(A) LENGTH: 59 base pairs
(B) TYPE: nucleic acid

(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:19:
GATATCTAAT ACGACTCACT ATAGGGAGAC TAGTGGCCAC
CATGTTCTTG TTAACAACT 59

(2) INFORMATION FOR SEQ ID NO:20:
(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:20:
GATATCTAAT ACGACTCACT ATAGGGAGAG GTATGGTACT
TGGTAGTT 48

(2) INFORMATION FOR SEQ ID NO:21:
(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:21:
TCTAATACGA CTCACTATAG GGAGA 25

## Description

### PRIORITY

The present invention claims priority to U.S. Provisional Patent Application No. 63/531,004 filed on August 6, 2023, which is entitled "Novel In-Cell Transcription (ICT) Composition and the Related Vaccine Medicine Designs Thereof'. The present invention also claims priority to U.S. Patent Application No. 17/489,357 filed on September 29, 2021, which is entitled "Novel mRNA Composition and Production Method for Use in Anti-Viral and Anti-Cancer Vaccines". Moreover, the present application is a continuation-in-part application of the U.S. Patent Application No. 17/489,357 filed on September 29, 2021, which is entitled "Novel mRNA Composition and Production Method for Use in Anti-Viral and Anti-Cancer Vaccines".

The instant application contains a Sequence Listing which has been submitted electronically in XML format and is hereby incorporated by reference in its entirety. Said XML copy, created on October 12, 2023, is named 23P234-EP_SL.xml and is 168,296 bytes in size.

### FIELD OF THE INVENTION

This invention relates to a novel non-vector-based (non-transgenic), induced cytoplasmic in-vitro transcription (ICIVT) composition useful for inducing in-vitro transcription (IVT)-like RNA/mRNA amplification in the target cells of interest after intracellular transfection in vitro, ex vivo as well as in vivo. The present invention is useful for designing and developing a variety of RNA/mRNA-based medicines and/or vaccines comprising a novel mixture composition of at least a designed promoter-linked RNA/mRNA-coding DNA (PLRcD) template and another DNA-dependent RNA polymerase (DdRP) mRNA sequence. Preferably, the DdRP mRNA is selected from the transcripts of T7, T3 and/or SP6 RNA polymerases, or a combination thereof, while the designed PLRcD template may encode at least a transcript or transcripts of antisense RNA oligonucleotide (aRNA-ASO), small interferring RNA (siRNA), double-stranded RNA (dsRNA), short hairpin RNA (shRNA), microRNA (miRNA)/microRNA precursor (pre-miRNA), long noncoding RNA (lncRNA), messenger RNA (mRNA), and/or self-amplifying RNA/mRNA (saRNA/samRNA), or a combination thereof. For delivery/transfection into the cells of interest, the PLRcD and DdRP mixture composition may be further mixed, conjugated, encapsulated or formulated with at least a reagent selected from, but not limited to, liposomes, nanoparticles, liposomal nanoparticles (LNP), exosomes, triglycylglycerin (TGG)-derived agents, sugar-/glucosamine-/galatosamine-based conjugating molecules, infusion/transfusion agents, gene gun materials, electroporation agents, and/or transposons/retrotransposons, or a combination thereof. After transfection into the target cells of interest, the expression of the resulting encoded RNA/mRNA is useful for treating a variety of human diseases, including but not limited to Alzheimer's disease, Parkinson's disease, motor neuron disease, stroke, diabetes, myocardial infraction, hemophilia, anemia, leukemia, and all sorts of cancers as well as many kinds of viral and bacterial infections.

### BACKGROUND OF THE INVENTION

Prior in-vitro transcription (IVT) technology is a method of using DNA-dependent RNA polymerase (DdRP) proteins to synthesize and amplify desired RNA/mRNA sequences derived from a pre-designed promoter-linked DNA template in test tubes in vitro, no involvement of any cell, cytoplasm, or DdRP mRNA. Hence, although based on a similar IVT principle, but with different compositions and practical mechanisms, prior IVT technologies are different from the in-cell, induced cytoplasmic IVT (ICIVT) methodology of the present invention.

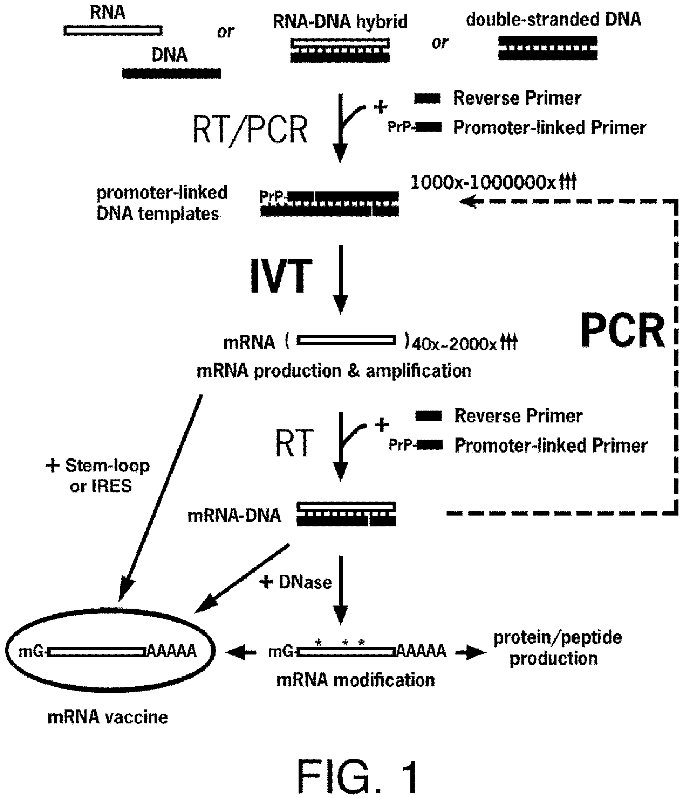

The use of in-vitro transcription (IVT) methods to produce sense (+) strand mRNAs and/or the related mRNA-cDNA hybrids was first reported by Lin et al (U.S. Patents No. 7,662,791, 8,080,652, 8,372,969, and 8,609,831 to Lin). As shown in FIG. 1, Lin's methods first use polymerase chain reaction (PCR) and/or reverse transcription (RT) to incorporate a specific RNA promoter-primer into the resulting DNA products, so as to generate promoter-linked mRNA-coding DNA templates suitable for IVT-based mRNA amplification (Lin et al, Methods Mol Biol. 221:93-101, 2003). After IVT and further RNase-free DNase digestion, the amplified mRNAs can be formulated with a delivery/transfection agent and then transfected into the target cells of interest for producing mRNA-encoded proteins/peptides. Yet, although Lin's methods had been successfully used to produce mRNAs and the related proteins/peptides in test tubes in vitro, there is no description of any IVT-like reaction or application in cells. Hence, Lin's prior IVT methods did not disclose any information related to the in-cell transcription of the present invention.

Today's mRNA production methods are still largely based on a part or whole of Lin's PCR-IVT protocol (U.S. Patents No. 7,662,791, 8,080,652, 8,372,969, and 8,609,831 to Lin). Also, many commercial semi- or fully-automated IVT devices had been designed and developed using Lin's IVT concept and methodology. Yet, these prior IVT-related methods and devices still did not provide any effective solution to overcome the problem of IVT activation in eukaryotic cells, particularly in mammalian cells, due to incompatibility of eukaryotic DdRP to prior IVT-related bacteriophage promoters (*i*.*e*. T7, T3 and SP6 promoters). Since prior IVT promoters are not compatible with eukaryotic DdRP enzymes, the transcription of a prior designed promoter-linked RNA/mRNA-coding DNA (PLRcD) sequence normally can not be activated in mammalian cells, leading to no in-cell RNA/mRNA amplification. To solve this problem, another method using plasmid/replicon-driven RNA/mRNA expression in prokaryotes (e.g. bacteria) had also been developed by Lin et al (U.S. Patents No. 9,637,747 and 9,783,811 to Lin). Nevertheless, this plasmid/replicon-driven RNA/mRNA expression method requires the use of prokaryotic cells and is only feasible in prokaryotes.

Transgenic and vector/replicon (*e*.*g*. plasmid)-based T7-DdRP-mediated IVT in mammalian cells had also been established (Ghaderi et al., Iran J. Cancer Prev. 7:137-141, 2014) In prior practice, a plasmid vector encoing T7 DdRP is transfected into the cell nucleus for expressing T7 DdRP mRNA as well as proteins and then the T7 DdRP proteins drive the expression of another co-transfected T7-promoter-linked mRNA-coding plasmid vector in the transfected cells. Yet, it is uncertain whether the second encoded mRNA is expressed in cell nucleus or cytoplasm because the expressed T7 DdRP may be presented in either one or both locations. It is also uncertain whether the co-transfected T7-promoter-linked mRNA-coding plasmid is delivered into cell nucleus or cytoplasm. As this vector-based IVT methodology requires intranuclear expression of T7 DdRP mRNA, it is surely contain a risk of transgenic insertion. Also, because the T7 DdRP mRNA is expressed by the mammalian transcription system in the cell nucleus, the involvement of another eukaryotic promoter driven by mammalian type-II RNA polymerases (Pol-II) is required as well. As a result, in view of such a complicated Pol-II+T7-driven mRNA expression mechanism, this vector (plasmid)-based IVT system in mammalian cells is not truly a cytoplasmic transcription system.

Following the fast development of mRNA vaccines and medicines today, it is desirable to induce non-transgenic IVT-like RNA/mRNA amplification in the cytoplasm of the transfected cells of interest. Yet, none of the prior IVT methods can achieve this goal. To overcome the problems of prior IVT methods, the presnt invention adopt a novel induced cytoplasmic IVT (ICIVT) composition and method for inducing specific IVT-like RNA/mRNA amplification in the transfected cells of interest, particularly in mammalian cells.

### SUMMARY OF THE INVENTION

The principle of the present invention is relied on the novel composition designs described in the claimed priority U.S. Patent Application No. 17/489,357, but more advaned. As shown in FIG. 2, by insertion of at least an internal ribosome entry site (IRES)-linked kozak motif (IRES-kozak) between the promoter and RNA/mRNA-coding sequences, a designed construct of induced cytoplasmic IVT (ICIVT)-based promoter-linked RNA/mRNA-coding DNA (PLRcD) template is herein generated, useful for inducing IVT-like RNA/mRNA amplification in the cytoplasm of the transfected cells of interest, leading to a cytoplasmic RNA/mRNA expression system. In order to activate this cytoplasmic RNA/mRNA expression system, we need to co-transfect mRNA of at least a DNA-dependent RNA polymerase (DdRP), such as T7, T3, and/or SP6 DdRP mRNA, with the designed PLRcD template. Yet, because it is uncertain whether a foreign mRNA (*e*.*g*. T7, T3, or SP6 DdRP mRNA) without going through any intranuclear processing and/or modification can still be translated into functional proteins, particularly with proper folding structures, in mammalian cells, it is understandable that even a skillful person in the field must perform extensive experiments and modifications to prove the feasibility of the designed DdRP mRNA for forming functional DdRP proteins in mammalian cytoplasm.

The kozak motif is a nucleotide sequence of 5'-RCCRCC-3' (R is A or G) or 5'-RCCDCC-3' (D is A, G or T/LT), while the IRES may contain at least one homologue of the following hairpin/stem-loop-containing sequences, of which the contents U (uracil/uridine) and T (thymine/thymidine) are exchangable, including but not limited to:

(1) 5'-GCUCCCUUCA ACUUUAACAU GGAAGUGCUU UCUGUGACUU
UAAAAGUAAG UGCUUCCAUG UUUUAGUAGG AGU-3' (73-nt) (SEQ ID
NO:3)

(2) 5’-CCUUUGCUUU AACAUGGGGG UACCUGCUGU GUGAAACAAA
AGUAAGUGCU UCCAUGUUUC AGUGGAGG-3’ (68-nt) (SEQ ID NO:4)

(3) 5'-CCACCACUUA AACGUGGAUG UACUUGCUUU GAAACUAAAG
AAGUAAGUGC UUCCAUGUUU UGGUGAUGG-3' (69-nt) (SEQ ID NO:5)

(4) 5’-CCUCUACUUU AACAUGGAGG CACUUGCUGU GACAUGACAA
AAAUAAGUGC UUCCAUGUUU GAGUGUGG-3’ (68-nt) (SEQ ID NO:6)

(5) 5'-CUGUGUGGCU GUCACUCGGC UGCAUGCUUA GUGCACUCAC
GCAG-3' (44-nt) (SEQ ID NO:7)

(6) 5'-CUGUGUGGCU GUCACUCGGC UGCAUGCUUA GUGCACUCAC
GCAGUAUAAU UAAUAACUAA UUACU-3' (65-nt) (SEQ ID NO:8)

(7) 5'-GUCGUUGACA GGACACGAGU AACUCGUCUA UCUUCUGCAG
GCUGCUUACG GUUUCGUCCG UGUUGCAGCC GAUCAUCAGC
ACAUCUAGGU UUCGUCCGGG UGUGACCGAA AGGUAAGAUG
GAGAGCCUUG UCCCUGGUUU CAACGAG-3' (147-nt) (SEQ ID NO:9)

(8) 5'-AAUUAUAAAU UACCAGAUGA UUUUACAGGC UGCGUUAUAG
CUUGGAAUUC UAACAAUCUU GAUUCUAAGG UUGGUGGUAA

UUAUAAUU-3’ (88-nt) (SEQ ID NO:10)

(9) 5'-CACAAAUAUU ACCAGAUCCA UCAAAACCAA GCAAGAGGUC
AUUUAUUGAA GAUCUACUUU UCAACAAAGU GACACUUGCA
GAUGCUGGCU UCAUCAAACA AUAUGGUGAU UGCCUUGGUG
AUAUUGCUG-3' (129-nt) (SEQ ID NO:11)

(10) 5'-GCAAAAUGU GAUCUUGCUU GUAAAUACAA UUUUGAGAGG
UUAAUAAAUU ACAAGUAGUG CUAUUUUUGU AUUUAGGUUA
GCUAUUUAGC UUUACGUUCC AGGAUGCCUA GUGGCAGCCC
CACAAUAUCC AGGAAGCCCU CUCUGCGGUU UUUCAGAUUC
GUUAGUCGAA AAACCUAAGA AAUUUAAUG-3' (189-nt) (SEQ ID NO:12)

(11) 5'-CACUCCCCUG UGAGGACUAC UGUCUUCACG CAGAAAGCGU
CUAGCCAUGG CGUUAGUAUG AGUGUCGUGC AGCCUCCAGG
ACCCCCCCUC CCGGGAGAGC CAUAGUGGUC UGCGGAACCG
GUGAGUACAC CGGAAUUGCC AGGACGACCG GGUCCUUUCU
UGGAUCAACC CGCUCAAUGC CUGGAGAUUU GGGCGUGCCC
CCGCGAGACU GCUAGCCGAG UAGUGUUGGG UCGCGAAAGG
CCUUGUGGUA CUGCCUGAUG GGUGCUUGCG AGUGCCCCGG
GAGGUCUCGU AGAC-3' (294-nt) (SEQ ID NO:13)

(12) 5'-GGACACGAGU AACUCGUCUA UCUUCUGCAG GCUGCUUACG
GUUUCGUCCG UGUUG-3' (55-nt) (SEQ ID NO:14)

(13) 5'-CAGCCGAUCA UCAGCACAUC UAGGUUUUGU CCGGGUGUGA
CCGAAAGGUA AG-3' (52-nt) (SEQ ID NO:15)

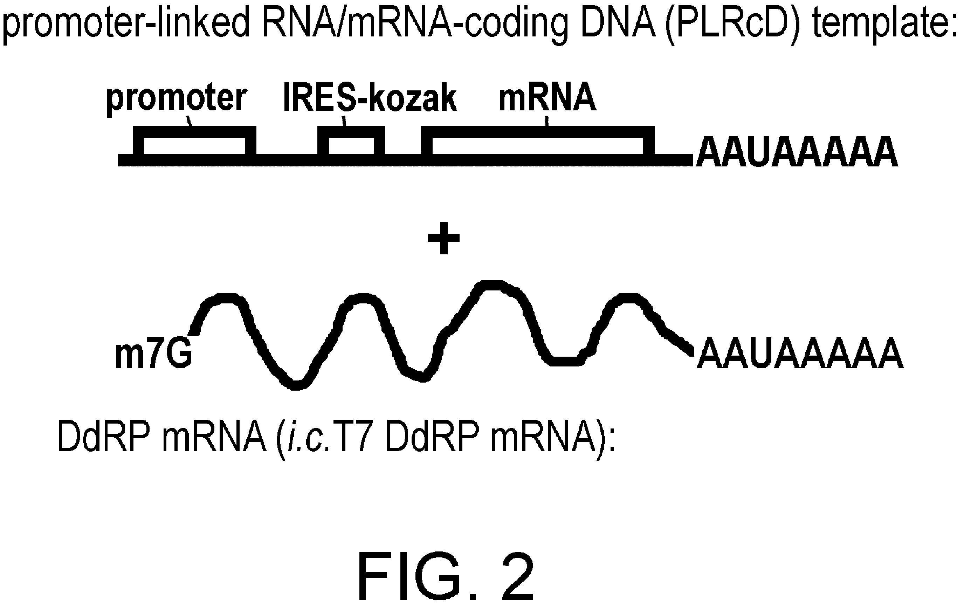

For performing induced cytoplasmic IVT-like transcription (ICIVT), we have invented a method of co-transfection of at least a man-made DdRP mRNA (*e.g.* T7, T3, and/or SP6 DdRP mRNA) and the designed PLRcD template(s) together into the target cells of interest. Also, since the ICIVT-amplified RNA/mRNA products so obtained (located in cytoplasm/cytosol) may not be effectively capped by intracellular capping enzymes (in nucleus), the currently invented IRES-kozak motif in the designed PLRcD template will then serve as an internal ribosome entry site (IRES) for ribosome assembly to activate protein/peptide translation, while the kozak sequence marks the precise position for initiating protein/peptide synthesis. Hence, due to our novel design of the IRES-kozak motif (FIG. 2), the presently invented ICIVT-based PLRcD templates can be effectively used for inducing not only the in-cell transcription (or called cytoplasmic expression) of desired RNA/mRNA sequences but also the translation of the resulting mRNA products into proteins/peptides in the transfected cells. Notably, the resulting RNA may be either coding mRNA or non-coding RNA, or a combination of both.

In order to prevent unneccessary interferon-associated immune response and intracellular Trex1-mediated DNA degradation , the designed PLRcD template is dephosphorylated in the 5'-end and tailed by 8-hydroxyguanine (8-OHG) and/or its derived analogs, particularly 8-hydroxy-2-deoxyguanosine (8-OHdG), in the 3'-end, respectively, while the DdRP mRNA is capped by m7G(5')ppp(5')N (m7G) and/or its related cap (*i.e*. cap-0 or cap-1/2) analogs in the 5'-end and may be further tailed by a cap-like modified nucleotide analog (*e*.*g*. 3'-cap modified G/U/A molecule, such as 8-oxo-G, 8-oxo-A or 5'phosphorothioate-U) in the 3'-end, respectively. For 5'-cap incorporation, the capping enzymes include, but not limited to, coronaviral NSP12, NSP9/14, NSP10/16, and/or vaccinia capping enzyme as well as 2'-O-methyltransferase, or a combination thereof. For 3'-cap incorporation, terminal RNA uridylyl transferases and/or polymerase theta are preferably used. Additionally, in order to prevent intracellular immune response, the U (uridine/uracil) contents of the DdRP mRNA can be further completely or partially replaced by pseudouridine, methyluridine, methoxyuridine, and/or other related or similarly modified nucleotide analogs, or a combination thereof.

For facilitating delivery/transfection into the target cells of interest in vitro, ex vivo and/or in vivo, the mixture composition of the designed PLRcD template and DdRP mRNA (FIG. 2) can be mixed, conjugated, encapsulated and/or formulated with at least a delivery/transfection agent selected from, but not limited to, liposomes, nanoparticles, liposomal nanoparticles (LNP), exosomes, sugar-/glucosamine-/galatosamine-based conjugating molecules, infusion/transfusion agents, glycylglycerin-derived molecules, gene gun materials, electroporation agents, and/or transposons/retrotransposons, or a combination thereof.

IRES is a highly structured nucleotide sequence, frequently containing harpin-like and/or stem-loop structures. For producing highly structured PLRcD templates, our claimed priority U.S. patent application No. 17/489,357 (`357) had developed a novel PCR-IVT methodology using a specific helicase activity for overcoming the low efficiency problem of highly structured RNA amplification in traditional IVT reactions. Because hairpin-like stem-loop structures are signals of intrinsic transcription termination for prokaryotic RNA polymerases (McDowell et al, Science 266:822-825, 1994), the claimed priority `357 method adopts a new IVT system with a mixture of RNA polymerase and helicase activities. As a result, this additional helicase activity markedly reduces the secondary structures of PLRcD templates and the resulting RNA/mRNA products in IVT, leading to a higher RNA/mRNA amplification rate. To this, based on our studies, coronaviral NSP7 and NSP13 proteins are two of the identified helicases useful for enhancing the amplification efficiency of IVT and ICIVT.

The advantages of using the presently invented novel composition of the designed PLRcD template and DdRP mRNA mixture include (1) ) in-cell RNA/mRNA amplification similar to the effect of self-amplifying RNA, (2) no transgene problem, (3) no necessity of 5'-cap addition in the resulting RNA/mRNA products, (4) long-lasting efficacy due to the stable DNA constructs of the designed PLRcD templates, (5) adjustable encoded RNA/mRNA expression modulated by the used amount of DdRP mRNA, (6) easy manufacturing procedure, (7) further enhancement by using self-amplifying DdRP mRNA (DdRP-samRNA), and (8) a variety of potential applications developed. Conceivably, the novel composition of the invented PLRcD template and DdRP mRNA mixture is useful for designing and developing new pharmaceutical and therapeutic applications, including vaccines and medicines, for use in treating a variety of human/animal diseases, inculding but not limited to Alzheimer's disease, Parkinson's disease, motor neuron disease, stroke, diabetes, myocardial infraction, hemophilia, anemia, leukemia, and many kinds of cancers as well as all sorts of viral and bacterial infections.

### A. Definitions

To facilitate understanding of the invention, a number of terms are defined below:
Nucleic Acid: a polymer of deoxyribonucleic acid (DNA) or ribonucleic acid (RNA), either single or double stranded.
Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. A nucleoside containing at least one phosphate group bonded to the 3' or 5' position of the pentose is a nucleotide. DNA and RNA are consisted of different types of nucleotide units called deoxyribonucleotide and ribonucleotide, respectively.
Deoxyribonucleoside Triphosphates (dNTPs): the building block molecules for DNA synthesis, including dATP, dGTP, dCTP, and dTTP and sometimes may further containing some modified deoxyribonucleotide analogs.
Ribonucleoside Triphosphates (rNTPs): the building block molecules for RNA synthesis, including ATP, GTP, CTP, and UTP and sometimes may further containing pseudouridine, 5'methyluridine, methoxyuridine, and/or some other modified ribonucleotide analogs.
Nucleotide Analog: a purine or pyrimidine nucleotide that differs structurally from adenine (A), thymine (T), guanine (G), cytosine (C), or uracil (U), but is sufficiently similar to substitute for the normal nucleotide in a nucleic acid molecule.
Oligonucleotide: a molecule comprised of two or more monomeric units of DNA and/or RNA, preferably more than three, and usually more than ten. An oligonucleotide longer than 13 nucleotide monomers is also called polynucleotiude. The exact size will depend on many factors, which in turn depends on the ultimate function or use of the oligonucleotide. The oligonucleotide may be generated in any manner, including chemical synthesis, DNA replication, RNA transcription, reverse transcription, or a combination thereof.
Nucleic Acid Composition: a nucleic acid composition refers to an oligonucleotide or polynucleotide such as a DNA or RNA sequence, or a mixed DNA/RNA sequence, in either a single-stranded or a double-stranded molecular structure.
Gene: a nucleic acid composition whose oligonucleotide or polynucleotide sequence codes for an RNA and/or a polypeptide (protein). A gene can be either RNA or DNA. A gene may encode a non-coding RNA, such as small hairpin RNA (shRNA), microRNA (miRNA), rRNA, tRNA, snoRNA, snRNA, and their RNA precursors as well as derivatives. Alternatively, a gene may encode a protein-coding RNA essential for protein/peptide synthesis, such as messenger RNA (mRNA) and its RNA precursors as well as derivatives. In some cases, a gene may encode a protein-coding RNA that also contains at least a microRNA or shRNA sequence.
Primary RNA Transcript: an RNA sequence that is directly transcribed from a gene without any RNA processing or modification.
Precursor messenger RNA (pre-mRNA): primary RNA transcripts of a protein-coding gene, which are produced by eukaryotic type-II RNA polymerase (Pol-II) machineries in eukaryotes through an intracellular mechanism termed transcription. A pre-mRNA sequence contains a 5'-untranslated region (UTR), a 3'-UTR, exons and introns.
Intron: a part or parts of a gene transcript sequence encoding non-protein-reading frames, such as in-frame intron, 5'-UTR and 3'-UTR.
Exon: a part or parts of a gene transcript sequence encoding protein-reading frames (cDNA), such as cDNA for cellular genes, growth factors, insulin, antibodies and their analogs/homologs as well as derivatives.
Messenger RNA (mRNA): assembly of pre-mRNA exons, which is formed after intron removal by intracellular RNA splicing machineries (*e*.*g*. spliceosomes) and served as a protein-coding RNA for peptide/protein synthesis. Structurally, mRNA sequence may comprise 5'-cap nucleotide [*e.g.* m7G(5')ppp(5')N-], 5'-untranslated region (5'-UTR), at least a Kozak consensus translation initiation site (*e*.*g*. 5'-GCCACC-3'), at least a protein/peptide-coding region, polyadenylation signals (*e*.*g*. 5'-AUAAA-3' or 5'-AUUAAA-3'), and/or 3'-UTR with or without poly(A) tail. The proteins/peptides encoded by mRNAs include, but not limited to, enzymes, growth factors, insulin, antibodies and their analogs/homologs as well as derivatives.
Complementary DNA (cDNA): a single-stranded or double-stranded DNA that contains a sequence complementary to an mRNA sequence and does not contain any intronic sequence.
Sense: a nucleic acid molecule in the same sequence order and composition as the homologous mRNA. The sense conformation is indicated with a "+", "s" or "sense" symbol.
Antisense: a nucleic acid molecule complementary to the respective mRNA molecule. The antisense conformation is indicated as a "-" symbol or with an "a" or "antisense" in front of the DNA or RNA, e.g., "aDNA" or "aRNA".
Base Pair (bp): a partnership of adenine (A) with thymine (T), or of cytosine (C) with guanine (G) in a double stranded DNA molecule. In RNA, uracil (U) is substituted for thymine. Generally the partnership is achieved through hydrogen bonding. For example, a sense nucleotide sequence "5'-A-T-C-G-U-3'" can form complete base pairing with its antisense sequence "5'-A-C-G-A-T-3'".
5'-end: a terminus lacking a nucleotide at the 5' position of successive nucleotides in which the 5'-hydroxyl group of one nucleotide is joined to the 3'-hydroyl group of the next nucleotide by a phosphodiester linkage. Other groups, such as one or more phosphates, may be present on the terminus.
3'-end: a terminus lacking a nucleotide at the 3' position of successive nucleotides in which the 5'-hydroxyl group of one nucleotide is joined to the 3'-hydroyl group of the next nucleotide by a phosphodiester linkage. Other groups, most often a hydroxyl group, may be present on the terminus.
Template: a nucleic acid molecule being copied by a nucleic acid polymerase. A template can be single-stranded, double-stranded or partially double-stranded, RNA or DNA, depending on the polymerase. The synthesized copy is complementary to the template, or to at least one strand of a double-stranded or partially double-stranded template. Both RNA and DNA are synthesized in the 5' to 3' direction. The two strands of a nucleic acid duplex are always aligned so that the 5' ends of the two strands are at opposite ends of the duplex (and, by necessity, so then are the 3' ends).
Nucleic Acid Template: a double-stranded DNA molecule, double-stranded RNA molecule, hybrid molecules such as DNA-RNA or RNA-DNA hybrid, or single-stranded DNA or RNA molecule.
Conserved: a nucleotide sequence is conserved with respect to a pre-selected (referenced) sequence if it non-randomly hybridizes to an exact complement of the pre-selected sequence.
Homologous or Homology: a term indicating the similarity between a polynucleotide and a gene or mRNA sequence. A nucleic acid sequence may be partially or completely homologous to a particular gene or mRNA sequence, for example. Homology may be expressed as a percentage determined by the number of similar nucleotides over the total number of nucleotides.
Complementary or Complementarity or Complementation: a term used in reference to matched base pairing between two polynucleotides (*e*.*g*. sequences of an mRNA and a cDNA) related by the aforementioned "base pair (bp)" rules. For example, the sequence "5'-A-G-T-3'" is complementary to not only the sequence "5'-A-C-T-3'" but also to "5'-A-C-U-3'". Complementation can be between two DNA strands, a DNA and an RNA strand, or between two RNA strands. Complementarity may be "partial" or "complete" or "total". Partial complementarity or complementation occurs when only some of the nucleic acid bases are matched according to the base pairing rules. Complete or total complementarity or complementation occurs when the bases are completely or perfectly matched between the nucleic acid strands. The degree of complementarity between nucleic acid strands has significant effects on the efficiency and strength of hybridization between nucleic acid strands. This is of particular importance in amplification reactions, as well as in detection methods that depend on binding between nucleic acids. Percent complementarity or complementation refers to the number of mismatch bases over the total bases in one strand of the nucleic acid. Thus, a 50% complementation means that half of the bases were mismatched and half were matched. Two strands of nucleic acid can be complementary even though the two strands differ in the number of bases. In this situation, the complementation occurs between the portion of the longer strand corresponding to the bases on that strand that pair with the bases on the shorter strand.
Complementary Bases: nucleotides that normally pair up when DNA or RNA adopts a double stranded configuration.
Complementary Nucleotide Sequence: a sequence of nucleotides in a single-stranded molecule of DNA or RNA that is sufficiently complementary to that on another single strand to specifically hybridize between the two strands with consequent hydrogen bonding.
Hybridize and Hybridization: the formation of duplexes between nucleotide sequences which are sufficiently complementary to form complexes via base pairing. Where a primer (or splice template) "hybridizes" with target (template), such complexes (or hybrids) are sufficiently stable to serve the priming function required by a DNA polymerase to initiate DNA synthesis. There is a specific, *i.e.* non-random, interaction between two complementary polynucleotides that can be competitively inhibited.
Posttranscriptional Gene Silencing: a targeted gene knockout or knockdown effect at the level of mRNA degradation or translational suppression, which is usually triggered by either foreign/viral DNA or RNA transgenes or small inhibitory RNAs.
RNA Interference (RNAi): a posttranscriptional gene silencing mechanism in eukaryotes, which can be triggered by small inhibitory RNA molecules such as microRNA (miRNA), small hairpin RNA (shRNA) and small interfering RNA (siRNA). These small RNA molecules usually function as gene silencers, interfering with expression of intracellular genes containing either completely or partially complementarity to the small RNAs.
MicroRNA (miRNA): single-stranded RNAs capable of binding to targeted gene transcripts that have partial complementarity to the miRNA. MiRNA is usually about 17-27 oligonucleotides in length and is able to either directly degrade its intracellular mRNA target(s) or suppress the protein translation of its targeted mRNA, depending on the complementarity between the miRNA and its target mRNA. Natural miRNAs are found in almost all eukaryotes, functioning as a defense against viral infections and allowing regulation of gene expression during development of plants and animals.
Precursor MicroRNA (Pre-miRNA): hairpin-like single-stranded RNAs containing stem-arm and stem-loop regions for interacting with intracellular RNaseIII endoribonucleases to produce one or multiple microRNAs (miRNAs) capable of silencing a targeted gene or genes complementary to the microRNA sequence(s). The stem-arm of a pre-miRNA can form either a perfectly (100%) or a partially (mis-matched) hybrid duplexes, while the stem-loop connects one end of the stem-arm duplex to form a circle or hairpin-loop conformation. In the present invention, however, precursor of microRNA may also includes pri-miRNA.
Small interfering RNA (siRNA): short double-stranded RNAs sized about 18-27 perfectly base-paired ribonucleotide duplexes and capable of degrading target gene transcripts with almost perfect complementarity.
Small or short hairpin RNA (shRNA): single-stranded RNAs that contain a pair of partially or completely matched stem-arm nucleotide sequences divided by an unmatched loop or bubble oligonucleotide to form a hairpin-like structure. Many natural miRNAs are derived from small hairpin-like RNA precursors, namely precursor microRNA (pre-miRNA).
Vector: a recombinant nucleic acid composition such as recombinant DNA (rDNA) capable of movement and residence in different genetic environments. Generally, another nucleic acid is operatively linked therein. The vector can be capable of autonomous replication in a cell in which case the vector and the attached segment is replicated. One type of preferred vector is an episome, *i.e*., a nucleic acid molecule capable of extrachromosomal replication. Preferred vectors are those capable of autonomous replication and expression of nucleic acids. Vectors capable of directing the expression of genes encoding for one or more polypeptides and/or non-coding RNAs are referred to herein as "expression vectors" or "expression-competent vectors". Particularly important vectors allow cloning of cDNA from mRNAs produced using a reverse transcriptase. A vector may contain components consisting of a viral or a type-II RNA polymerase (Pol-II or pol-2) promoter, or both, a Kozak consensus translation initiation site (such as 5'-GCCACC-3'), polyadenylation signals (such as 5'-AUAAA-3' or 5'-AUUAAA-3'), a plurality of restriction/cloning sites, a pUC origin of replication, a SV40 early promoter for expressing at least an antibiotic resistance gene in replication-competent prokaryotic cells, an optional SV40 origin for replication in mammalian cells, and/or a tetracycline responsive element. The structure of a vector can be a linear or circular form of single- or double-stranded DNA selected form the group consisting of plasmid, viral vector, transposon, retrotransposon, DNA transgene, jumping gene, and a combination thereof.
Promoter: a nucleic acid to which a polymerase molecule recognizes, perhaps binds to, and initiates RNA transcription. For the purposes of the instant invention, a promoter can be a known polymerase binding site, an enhancer and the like, any sequence that can initiate synthesis of RNA transcripts by a desired polymerase.
RNA Processing: a cellular mechanism responsible for RNA maturation, modification and degradation, including RNA splicing, intron excision, exosome digestion, nonsense-mediated decay (NMD), RNA editing, RNA processing, 5'-capping, 3'-poly(A) tailing, and a combination thereof.
Gene Delivery: a genetic engineering method selected from the group consisting of polysomal transfection, liposomal transfection, chemical (nanoparticle) transfection, electroporation, viral infection, DNA recombination, transposon insertion, jumping gene insertion, microinjection, gene-gun penetration, and a combination thereof.
Genetic Engineering: a DNA recombination method selected from the group consisting of DNA restriction and ligation, homologous recombination, transgene incorporation, transposon insertion, jumping gene integration, retroviral infection, and a combination thereof.
Transfected Cell: a single or a plurality of eukaryotic cells after being artificially inserted with at least a nucleic acid sequence or protien/peptide molecule into the cell(s), selected from the group consisting of a somatic cell, a tissue cell, a stem cell, a germ-line cell, a tumor cell, a cancer cell, a virus-infected cell, and a combination thereof.
Antibody: a peptide or protein molecule having a pre-selected conserved domain structure coding for a receptor capable of binding a pre-selected ligand.
Pharmaceutical and/or therapeutic Application: a biomedical utilization and/or apparatus useful for stem cell generation, drug/vaccine development, non-transgenic gene therapy, cancer therapy, disease treatment, wound healing, tissue/organ repair and regeneration, and high-yield production of proteins/peptides/antibodies, drug ingredients, medicines, vaccines and/or food supplies, and a combination thereof.

### B. Compositions and Applications

A novel mixture composition of at least a promoter-linked RNA/mRNA/samRNA-coding DNA (PLRcD) template and at least a DNA-dependent RNA polymerase (DdRP) mRNA, wherein said PLRcD template contains at least an internal ribosome entry site (IRES)-linked kozak motif (IRES-kozak) located between the promoter and encoded RNA/mRNA/samRNA sequences.

The encoded RNA/mRNA/samRNA sequence may further contain at least a poly-A signal and/or poly-A tail. Also, the encoded RNA/mRNA/samRNA can be either protein-, peptide-, and/or antibody-coding mRNA, self-amplifying RNA/mRNA (saRNA/samRNA), or non-coding siRNA, shRNA, IncRNA, and/or microRNA (miRNA)/pre-miRNA, or a combination thereof. For increasing structural stability as well as for preventing intracellular immune response, the uridine/uracil (U) contents of the DdRP mRNA sequences can be totally or partially replaced by pseudouridine, methyluridine, methoxyuridine, or other related modified nucleotide analogs. For preventing unneccessary interferon-associated immune response as well as intracellular Trex1-mediated DNA degradation , the designed PLRcD template is dephosphorylated in the 5'-end and tailed by 8-hydroxyguanine (8-OHG) and/or its derived analogs (*e*.*g*. 8-OHdG) in the 3'-end, respectively, while the DdRP mRNA is capped by m7G(5')ppp(5')N (m7G) and/or its related 5'-cap analogs (*i.e*. cap-0, cap-1, and/or cap-2) in the 5'-end and may be further tailed by a cap-like modified nucleotide analog (*e*.*g*. 3'-cap modified G/U/A molecule, such as 8-oxo-G, 8-oxo-A and/or 5'-phosphorothioate-U) in the 3'-end, respectively. Moreover, the resulting RNA/mRNA/samRNA products may also comprise a 5'-cap molecule, such as m7G and/or its related cap analogs, preferably added and/or modified by vaccinia capping enzymes, coronaviral NSP12, NSP9/14, and/or NSP10/16, or a combination thereof. Furthermore, the mixture composition of the designed PLRcD template and DdRP mRNA may preferably further comprises coronaviral NSP7 and/or NSP13 helicase mRNA for enhancing the RNA/mRNA/ samRNA amplification by ICIVT. Taken together, the mixture composition of the designed PLRcD template and DdRP mRNA may additionally further comprises mRNA of NSP7, NSP13, NSP12, NSP9/14, and/or NSP10/16, or a combination thereof. For intracellular delivery, the mixture composition of the designed PLRcD template and DdRP mRNA can be further mixed, conjugated, encapsulated or formulated with at least a delivery/transfection agent selected from, but not limited to, liposomes, nanoparticles, liposomal nanoparticles (LNP), exosomes, sugar-/glucosamine-/galatosamine-based conjugating molecules, infusion/transfusion agents, triglycylglycerin (TGG)-derived molecules, gene gun materials, electroporation agents, and/or transposons/retrotransposons, or a combination thereof.

### BRIEF DESCRIPTION OF THE DRAWINGS

Referring particularly to the drawings for the purpose of illustration only and not limitation, there is illustrated:
FIG. 1 depicts the step-by-step procedure of the prior Lin's PCR-IVT methodology. For RNA production, a part or whole procedure of this PCR-IVT protocol may be adopted for either single or multiple cycle amplification of a desired RNA/mRNA sequence.
FIG. 2 depicts the structures of a designed promoter-linked RNA/mRNA-coding DNA (PLRcD) template and a DdRP mRNA. For enhancing DdRP mRNA stability, all or a part of the uridine/uracil (U) contents of the DdRP mRNA sequence can be replaced by pseudouridine, methyluridine, methoxyuridine, and/or other related or similarly modified nucleotide analogs. Also, the DdRP mRNA may be self-amplifying RNA/mRNA (saRNA/samRNA).

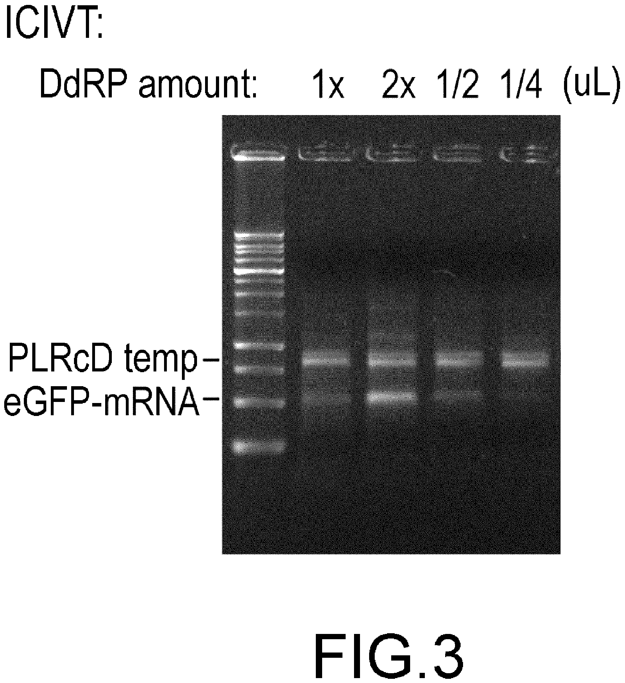

FIG. 3 shows the nondenaturing agarose gel electrophoresis results of IVT-like reactions using the mixture composition of a designed PLRcD template (*e*.*g*.
PLRcD encoding eGFP mRNA) and T7 DdRP. Notably, the expression level of encoded mRNA (*e*.*g*. eGFP mRNA) is regulated by the co-transfected amount of the used DdRP.
FIG. 4 shows microscopic examination results of the resulting eGFP protein expression induced by LNP-mediated co-transfection of the mixture composition of the designed PLRcD template (from FIG. 3) and T7 DdRP mRNA into target cells.

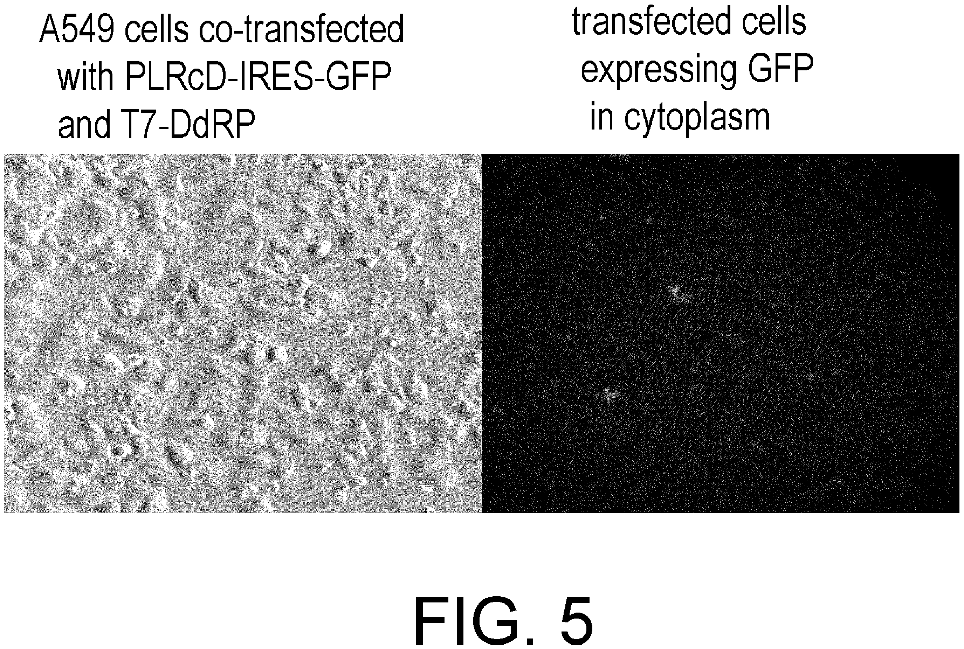

FIG. 5 shows another microscopic examination results of the resulting eGFP protein expression induced by electroporation of the mixture composition of another designed PLRcD template (modified from a commercial T7-promoter-driven IRES-eGFP plasmid vector) and T7 DdRP into target cells.

### DETAILED DESCRIPTION OF THE PREFERRED EMBODIMENTS

### EXAMPLES:

### 1. Human Cell Isolation and Cultivation

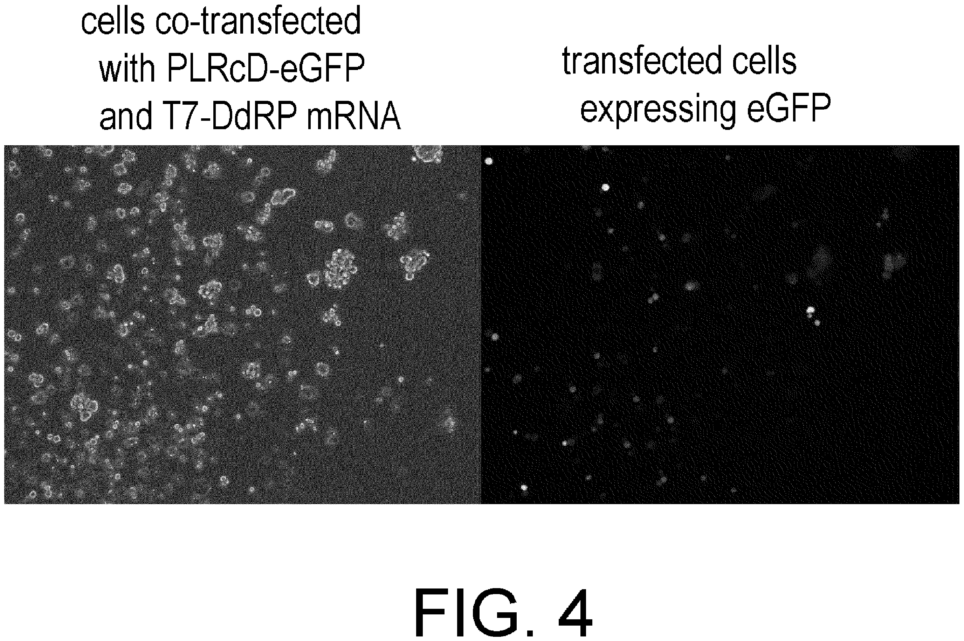

Human cancer and normal cell lines MCF7, HepG2, A549 and BEAS-2B were obtained either from the American Type Culture Collection (ATCC, Rockville, MD) or our collaborators and then maintained according to individual manufacturer's suggestions. For intracellular transfection, 0.5-500 µg of the mixture composition of PLRcD template and DdRP mRNA is mixed with 0.5 mL of fresh cell culture medium with addition of 1-50 µl of an In-VivoJetPEI transfection reagent, respectively. After 1-30 min incubation, the mixture is added into a cell culture containing 50%-60% confluency of the cultivated cells. The cell culture medium is reflashed every 12 to 48 hours, depending on the transfected cell types. This intracellular transfection procedure may be performed repeatedly to increase transfection efficiency. The transfection result is shown in FIG. 4.

### 2. In-Vivo Delivery/Transfection

The composition of a designed PLRcD template and DdRP mRNA mixture (weight ratio ranged from about 200:1 to 1:200) is mixed with a proper amount of a delivery/transfection reagent, such as In-VivoJetPEI agent or other similar LNP-based delivery/transfection reagents, following the manufacturer's protocols, and then injected into blood veins or muscles of an animal, depending on the purpose of involved applications. The delivery/transfection reagent is used for mixing, conjugating, encapsulating and/or formulating the designed mixture composition, so as to not only protect the DNA/mRNA contents from degradation but also facilitate the delivery/transfection of the mixture composition into specific target cells of interest in vitro, ex vivo as well as in vivo.

### 3. Preparation of PLRcD Templates and DdRP mRNA

Reverse transcription (RT) of desired RNA/mRNA is performed by adding about 0.01 ng~10 microgram (µg) of isolated RNA/mRNA into a 20-50 µL RT reaction (SuperScript III cDNA RT kit, ThermoFisher Scientific, MA, USA), following the manufacturer's suggestions. Depending on the RNA/mRNA amount, the RT reaction mixture further contains about 0.01-20 nmole RT primer, 0.1-10 mM each of deoxyribonucleoside triphosphate molecules (dNTPs; dATP, dTTP, dGTP and dCTP) and reverse transcriptase in 1x RT buffer. Then, the RT reaction is incubated at 37~65°C for 1-3 hours (hr), depending on the length and structural complexity of the desired RNA/mRNA sequence(s), so as to form the complementary DNA (cDNA) of the desired RNA/mRNA which is then used for the next step of PCR. Regarding RT primer designs, for demonstration but not limited to this design, we used 5'-CAGTTCCAAT TGTGAAGATT CTC-3' (SEQ ID NO: 16) for RT of a desired RdRp mRNA sequence and used another 5'-CTTGATGACG TTCTCAGTGC-3' (SEQ.ID.NO.17) for RT of another microRNA (miRNA)-containing green fluorescent protein (GFP)-coding mRNA (*e*.*g*. eGFP-coding mRNA) of interest, respectively.

Next, polymerase chain reaction (PCR) is performed by adding about 0.01 pg~10 µg of the RT-derived cDNAs in a 20-50 µL PCR preparation mixture (High-Fidelity PCR master kit, ThermoFisher Scientific, MA, USA), following the manufacturer's suggestion. Then, the PCR mixture is incubated in twenty to thirty (20~30) cycles of denaturation at 94°C for 30 sec-1 mim, annealing at 50~58°C for 30 sec-1 min, and then extension at 72°C for 1-3 min, depending on the structure and length of the desired cDNA sequences, respectively. For demonstration but not limited to this example, we used two sets of PCR primer pairs for amplifying two COVID-19 virus-associated PLRcD templates, including a pair of 5'-GATATCTAAT ACGACTCACT ATAGGGAGAG GTGCCACCAT GGTACTTGGT AGTT-3' (SEQ.ID.NO.18) and SEQ.ID.NO.16 (for amplifying an about 12.5k-nucleotide (nt) coronaviral RdRp/helicase/S protein-coding RNA) and another pair of 5'-GATATCTAAT ACGACTCACT ATAGGGAGAC TAGTGGCCAC CATGTTCTTG TTAACAACT-3' (SEQ.ID.NO.19) and SEQ.ID.NO.16 (for amplifying an about 2.8k-nt coronaviral S protein-coding mRNA). Moreover, we used another different pair of PCR primers for amplifying miRNA-containing GFP-coding PLRcD templates (*e*.*g*. the eGFP-coding mRNA), including a 5'-primer 5'-GATATCTAAT ACGACTCACT ATAGGGAGAG GTATGGTACT TGGTAGTT-3' (SEQ.ID.NO.20) and SEQ.ID.NO.17. In principle, the 5'-forward primers encode at least a conserved promoter sequence for IVT, including but not limited to T7, T3, and/or SP6 promoter sequences, particularly 5'-TCTAATACGA CTCACTATAG GGAGA-3' (SEQ.ID.NO.21).

For RNA (*e.g.* DdRP mRNA) production, since a promoter has been incorporated into the resulting PLRcD templates, a novel IVT reaction is prepared by using a mixture of 0.01 ng~10 µg of the PCR product, 0.1-10 U of helicase (*e*.*g*. coronaviral NSP7 and/or NSP13 proteins; optional), NTPs, and RNA polymerase (*i.e*. T7, T3, and/or SP6) in 1x transcription buffer. Then, the IVT reaction is incubated at 37°C for 1-6 hr, depending on the stability and activity of the used RNA polymerase(s). The resulting mRNA (*e*.*g*. DdRP mRNA) products are preferably further capped by using either vaccina cap enzymes, coronaviral NSP9/14, and/or NSP10/16 proteins, or a combination thereof. Preferably, the mRNA of NSP7, NSP13, NSP9/14 and/or NSP10/16, or a combination thereof, can also be used in the mixture composition of the designed PLRcD template and DdRP mRNA for intracellular co-transfection, so as to amplify 5'-capped mRNA in the transfected cells.

### 4. RNA Purification and Northern Blot Analysis

Desired RNAs (10 µg) are isolated with a *mir*Vana^{™} RNA isolation kit (Ambion, Austin, TX) or similar purification filter column, following the manufacturer's protocol, and then further purified by using either 5%~10% TBE-urea polyacrylamide or 1%~3.5% low melting point agarose gel electrophoresis. For Northern blot analysis, the gel-fractionated RNAs are electroblotted onto a nylon membrane. Detection of the RNA and its IVT template (the PCR-derived cDNA product) is performed with a labeled [LNA]-DNA probe complementary to a target sequence of the desired RNA. The probe is further purified by high-performance liquid chromatography (HPLC) and tail-labeled with terminal transferase (20 units) for 20 min in the presence of either a dye-labeled nucleotide analog or [³²P]-dATP (> 3000 Ci/mM, Amersham International, Arlington Heights,IL).

### 5. Protein Extraction and Western Blot Analysis

Cells (10⁶) are lysed with a CelLytic-M lysis/extraction reagent (Sigma) supplemented with protease inhibitors, Leupeptin, TLCK, TAME and PMSF, following the manufacturer's suggestion. Lysates are centrifuged at 12,000 rpm for 20 min at 4°C and the supernatant is recovered. Protein concentrations are measured using an improved SOFTmax protein assay package on an E-max microplate reader (Molecular Devices, CA). Each 30 µg of cell lysate are added to SDS-PAGE sample buffer under reducing (+50 mM DTT) and non-reducing (no DTT) conditions, and boiled for 3 min before loading onto a 6-8% polyacylamide gel. Proteins are resolved by SDS-polyacrylamide gel electrophoresis (PAGE), electroblotted onto a nitrocellulose membrane and incubated in Odyssey blocking reagent (Li-Cor Biosciences, Lincoln, NB) for 2 hr at room temperature. After that, a primary antibody is applied and mixed to the reagent and then together incubated at 4°C. After overnight incubation, the membrane is rinsed three times with TBS-T and then exposed to goat anti-mouse IgG conjugated secondary antibody to Alexa Fluor 680 reactive dye (1:2,000; Invitrogen-Molecular Probes), for 1 hr at the room temperature. After three additional TBS-T rinses, fluorescent scanning of the immunoblot and image analysis are conducted using Li-Cor Odyssey Infrared Imager and Odyssey Software v.10 (Li-Cor).

### 6. Immunostaining Assay

Cell/Tissue samples are first fixed in 100% methanol for 30 min at 4°C and then 4% paraformaldehyde (in 1x PBS, pH 7.4) for 10 min at 20°C. After that, the samples are further incubated in 1x PBS containing 0.1%-0.25% Triton X-100 for 10 min and then washed in 1x PBS three times for 5 min. For immunostaining, corresponding primary antibodies were purchased from Invitrogen (CA, USA) and Sigma-Aldrich (MO, USA), respectively. Dye-labeled goat anti-rabbit or horse anti-mouse antibody are used as the secondary antibody (Invitrogen, CA, USA). Results are examined and analyzed at 100x or 200x magnification under a fluorescent 80i microscopic quantitation system with a Metamorph imaging program (Nikon).

### 7. Statistic Analysis

All data were shown as averages and standard deviations (SD). Mean of each test group was calculated by AVERAGE of Microsoft Excel. SD was performed by STDEV Statistical analysis of data was performed by One-Way ANOVA. Tukey and Dunnett's t post hoc test were used to identify the significance of data difference in each group. *p* <0.05 was considered significant (SPSS v12.0, Claritas Inc).

### REFERENCES:

1. U.S. Patent No. 7,662,791 to Shi-Lung Lin et al.
2. U.S. Patent No. 8,080,652 to Shi-Lung Lin et al.
3. U.S. Patent No. 8,372,969 to Ying SY and Shi-Lung Lin.
4. U.S. Patent No. 8,609,831 to Shi-Lung Lin and Ying SY
5. U.S. Patent No. 9,637,747 to Shi-Lung Lin et al.
6. U.S. Patents No. 9,783,811 to Shi-Lung Lin et al.
7. U.S. Provisional Patent Application No. 60/222,479 to Shi-Lung Lin.
8. U.S. Provisional Patent Application No. 60/290,902 to Shi-Lung Lin.
9. Shi-Lung Lin and Ji H; Replicase cycling reaction amplification. WO2002/092774.
10. Shi-Lung Lin; Peptide library construction from RNA-PCR-derived RNAs. Methods Mol Biol. 221:289-293, 2003.
11. Shi-Lung Lin and Ji H; cDNA library construction using in-vitro transcriptional amplification. Methods Mol Biol. 221:93-101, 2003.
12. McDowell et al.; Determination of intrinsic transcription termination efficiency by RNA polymerase elongation rate. Science 266:822-825, 1994.
13. Ghaderi et al.; Construction of an eGFP expression plasmid under control of T7 promoter and IRES sequence for assay of T7 RNA polymerase activity in mammalian cell lines. Iran J. Cancer Prev. 7:137-141, 2014.

## Claims

1. A novel mixture composition of at least a promoter-linked RNA-coding DNA (PLRcD) template and at least a DNA-dependent RNA polymerase (DdRP) mRNA, wherein said PLRcD template contains at least an internal ribosome entry site (IRES)-linked kozak motif (IRES-kozak) located between the promoter and the encoded RNA sequences.

2. The composition as defined in Claim 1, wherein said mixture composition may further comprises mRNA of NSP7, NSP12, NSP13, NSP9/14, and/or NSP10/16 proteins, or a combination thereof.

3. The composition as defined in Claim 1, wherein said RNA encoded in the PLRcD template is non-coding RNA.

4. The composition as defined in Claim 1, wherein said RNA encoded in the PLRcD template is protein-/peptide- or antibody-coding mRNA.

5. The composition as defined in Claim 1, wherein said RNA encoded in the PLRcD template is self-amplifying RNA/mRNA (saRNA/samRNA).

6. The composition as defined in Claim 1, wherein said RNA encoded in the PLRcD template further contains at least a poly-A signal or poly-A tail.

7. The composition as defined in Claim 1, wherein said RNA encoded in the PLRcD template is a pharmaceutical compound or composition.

8. The composition as defined in Claim 1, wherein the 5'-end of said promoter-linked RNA-coding DNA (PLRcD) template is dephosphorylated.

9. The composition as defined in Claim 1, wherein the 3'-end of said promoter-linked RNA-coding DNA (PLRcD) template is further tailed by 8-hydroxyguanine (8-OHG) or its derived analogs, particularly 8-hydroxy-2-deoxyguanosine (8-OHdG).

10. The composition as defined in Claim 1, wherein said promoter of the PLRcD template is a bacteriophage RNA promoter, including T7, T3 and SP6 promoter.

11. The composition as defined in Claim 1, wherein said DNA-dependent RNA polymerase (DdRP) mRNA is the mRNA of a bacteriophage RNA polymerase, including T7, T3 and SP6 RNA polymerase.

12. The composition as defined in Claim 1, wherein the 5'-end of said DNA-dependent RNA polymerase (DdRP) mRNA is capped by a 5'-cap molecule.

13. The composition as defined in Claim 1, wherein the 3'-end of said DNA-dependent RNA polymerase (DdRP) mRNA is further tailed by a cap-like modified nucleotide analog.

14. The composition as defined in Claim 1, wherein said DNA-dependent RNA polymerase (DdRP) mRNA further contains modified nucleotide analogs.

15. The composition as defined in Claim 1, wherein the U (uridine/uracil) content of said DdRP mRNA is further completely or partially replaced by pseudouridine, methyluridine, methoxyuridine, and/or other similarly modified nucleotide analogs, or a combination thereof.

16. The composition as defined in Claim 1, wherein said promoter-linked RNA-coding DNA (PLRcD) template is produced using polymerase chain reaction (PCR).

17. The composition as defined in Claim 1, wherein said DNA-dependent RNA polymerase (DdRP) mRNA is produced using polymerase chain reaction and in-vitro transcription (PCR-IVT).

18. The composition as defined in Claim 1, wherein said mixture composition of at least a PLRcD template and at least a DdRP mRNA is further formulated with at least a delivery agent for facilitating intracellular transfection in vitro, ex vivo as well as in vivo.

19. The composition as defined in Claim 18, wherein said delivery agent includes liposomes, nanoparticles, liposomal nanoparticles (LNP), exosomes, conjugating molecules, infusion/transfusion agents, triglycylglycerin (TGG)-derived molecules, electroporation agents, and transposons/retrotransposons, or a combination thereof.

20. The composition as defined in Claim 1, wherein said internal ribosome entry site (IRES)-linked kozak motif (IRES-kozak) contains at least a SEQ ID NO:1 or SEQ ID NO:2.

21. The composition as defined in Claim 1, wherein said IRES-kozak motif contains at least an IRES sequence in the 5-end region of the kozak motif.

22. The composition as defined in Claim 21, wherein said IRES is selected from SEQ ID NOG, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, and SEQ ID NO: 13.

23. The composition as defined in Claim 1, wherein said IRES-kozak motif is incorporated into the PLRcD template using PCR.

24. The composition as defined in Claim 1, wherein said PLRcD template is a pharmaceutical compound or composition.

25. The composition as defined in Claim 1, wherein said DdRP mRNA is self-amplifying RNA/mRNA (saRNA/samRNA).

26. The composition as defined in Claim 1, wherein said DdRP mRNA is a pharmaceutical compound or composition.
